# EUROPEAN PATENT SPECIFICATION

(11) **EP 3 523 416 B1**
(45) Date of publication and mention of the grant of the patent: **19.02.2025**
(21) Application number: 17859189.7
(22) Date of filing: 05.10.2017
(51) Int. Cl.: C12N 1/15, C12N 1/19, C12N 1/21, C12N 5/078, C12N 5/10, C12N 15/09

(54) **METHODS AND COMPOSITIONS RELATED TO NK CELL AND ANTI-PDL1 CANCER THERAPIES**
VERFAHREN UND ZUSAMMENSETZUNGEN IN ZUSAMMENHANG MIT NK-ZELL- UND ANTI-PDL1-KREBSTHERAPIEN
MÉTHODES ET COMPOSITIONS ASSOCIÉES À DES THÉRAPIES ANTICANCÉREUSES DE CELLULES NK ET ANTI-PDL1

(30) Priority: 05.10.2016 US 201662404520 P
(43) Date of publication of application: 14.08.2019
(73) Proprietor: University of Central Florida Research Foundation, Inc., Orlando, FL 32826 (US)
(72) Inventor: COPIK, Alicja J., Orlando, Florida 32826 (US); IGARASHI, Robert Y., Orlando, Florida 32826 (US); OYER, Jeremiah J., Orlando, Florida 32826 (US); ALTOMARE, Deborah A., Orlando, Florida 32826 (US)
(74) Representative: Dehns
(86) International application number: PCT/US2017/055352
(87) International publication number: WO 2018/067825

(56) References cited:
- WO-A1-2016/134284
- WO-A2-2016/149201
- US-A1- 2015 216 937
- US-A1- 2015 359 853
- B. BOYERINAS ET AL: "Antibody-Dependent Cellular Cytotoxicity Activity of a Novel Anti-PD-L1 Antibody Avelumab (MSB0010718C) on Human Tumor Cells", CANCER IMMUNOLOGY RESEARCH, vol. 3, no. 10, 26 May 2015 (2015-05-26), US, pages 1148 - 1157, XP055389536, ISSN: 2326-6066, DOI: 10.1158/2326-6066.CIR-15-0059
- JEREMIAH L. OYER ET AL: "PD-L1 blockade enhances anti-tumor efficacy of NK cells", ONCOIMMUNOLOGY, vol. 7, no. 11, 27 August 2018 (2018-08-27), pages e1509819, XP055615840, DOI: 10.1080/2162402X.2018.1509819
- JEREMIAH L. OYER ET AL: "Natural killer cells stimulated with PM21 particles expand and biodistribute in vivo: Clinical implications for cancer treatment", CYTOTHERAPY, vol. 18, no. 5, 1 May 2016 (2016-05-01), GB, pages 653 - 663, XP055499303, ISSN: 1465-3249, DOI: 10.1016/j.jcyt.2016.02.006
- OYER ET AL.: "Natural Killer Cells Stimulated with PM21 Particles Expand and Biodistribute In Vivo: Clinical Implications for Cancer Treatment", CYTOTHERAPY, vol. 18, no. Iss. 5, 1 May 2016 (2016-05-01), pages 653 - 663, XP055499303
- OYER ET AL.: "Generation of Highly Cytotoxic Natural Killer Cells for Treatment of Acute Myelogenous Leukemia Using a Feeder-Free, Particle-Based Approach", BIOLOGY OF BLOOD AND MARROW TRANSPLANTATION, vol. 21, no. 4, 6 January 2015 (2015-01-06), pages 632 - 639, XP029176872

## Description

### I. BACKGROUND

1. It is now well established that expression of PD-L1 on most tumors is induced in response to IFNγ secreted by CD8 T cells recruited to the tumor site. Induction of PD-L1 expression on tumor initiates cascade of events such as the proliferation of regulatory T cells, upregulation of indoleamine-2,3-dioxygenase (IDO) that creates an immunosuppressive environment. PD-L1 also directly blocks the function of cytotoxic T cells through interaction with PD-1 receptor and leads to their anergy and apoptosis. These changes then aid tumor progression and metastasis.

2. Cancer therapies utilizing antibodies to disrupt PD-1/PD-L1 interaction have been among the most exciting developments so far leading to long lasting, durable remission in patients with various malignancies and even with very advanced disease. WO 2016/149201 discloses antibodies that bind to PD-L1, particularly activatable anti-PD-L1 antibodies and their use in therapy and diagnostics, particularly in combination with chimeric and pro-chimeric antigen receptor-modified NK cells. Boyerinas et al. 2015 (Cancer Immunology Research, vol. 3 (10), pp. 1148-1157) describes a study investigating the antibody-dependent cellular cytotoxicity activity of an anti-PD-L1 antibody, Avelumab, on human tumor cells. The new anti-PD-L1/anti-PD-1 therapies were able to unleash the paralyzed immune system in cancers where the initial immune response to tumor was halted by the induction of PD-L1 and the subsequent immunosuppressive cascade. Yet, responses are observed only in patients with PD-L1 positive tumors that are infiltrated with lymphocytes which is limited to 10-20% of patients, across tumor types. Thus PD-L1/PD-1 blockade therapies is only effective where there is induction of PD-L1 upon a strong initial response by the immune system. What is needed are new cancer therapies that can circumvent the PD-L1 immunosuppressive cascade, but are not limited to cancers where PD-L1 has been induced.

3. WO 2016/134284 discloses chimeric antigen receptors (CARs) comprising an antigen binding domain and/or a cytoplasmic domain of a IL-15 receptor α, and/or a chimeric cytoplasmic domain including a CD27 cytoplasmic domain fused to a 4-1BB cytoplasmic domain. The CARs may be expressed in NK cells.

4. US 2015/0359853 describes the use of complexes comprising IL-15 and IL-15 receptor α in therapy, such as to enhance IL-15-mediated immune function. It also discloses IL-15 responsive immune cells and their use in therapy, such as to treat cancer.

5. Oyer et al., 2016 (Cytotherapy, vol. 18, pp. 653-663) describes a particle-based method for expanding cytotoxic NK cells from peripheral blood mononuclear cells (PBMCs) using particles prepared from plasma membranes of K562-mb21-41BBL cells, expressing 41BBL and membrane bound IL-21.

### II. SUMMARY

6. Disclosed is an anti-PDL1 antibody and a component comprising activated NK cells for use in treating a cancer or an infection in a subject, wherein the activated NK cells are obtained by contacting a naive NK cell population with one or more of PM21 particles, EX21 exosomes and FC21 feeder cells.

7. In one aspect the NK cells may be derived from peripheral blood, umbilical cord blood, placental blood, from a CMV seropositive donor, or derived from other sources such as induced pluripotent stem cells, embryonic stem cells, or immortalized NK cell like cell lines such, as NK92.

8. The naive NK cells may be contacted *ex vivo* with one or more of IL-12, IL-15, and IL-18 and then contacted with one or more of PM21 particles, EX21 exosomes and FC21 feeder cells and/or the component further comprises one or more of IL-12, IL-15, and IL-18.

9. The activated NK cell component may further comprise PM21 particles, EX21 exosomes and/or FC21 cells.

### III. BRIEF DESCRIPTION OF THE DRAWINGS

10. The accompanying drawings, which are incorporated in and constitute a part of this specification, illustrate several embodiments and together with the description illustrate the disclosed compositions and methods.

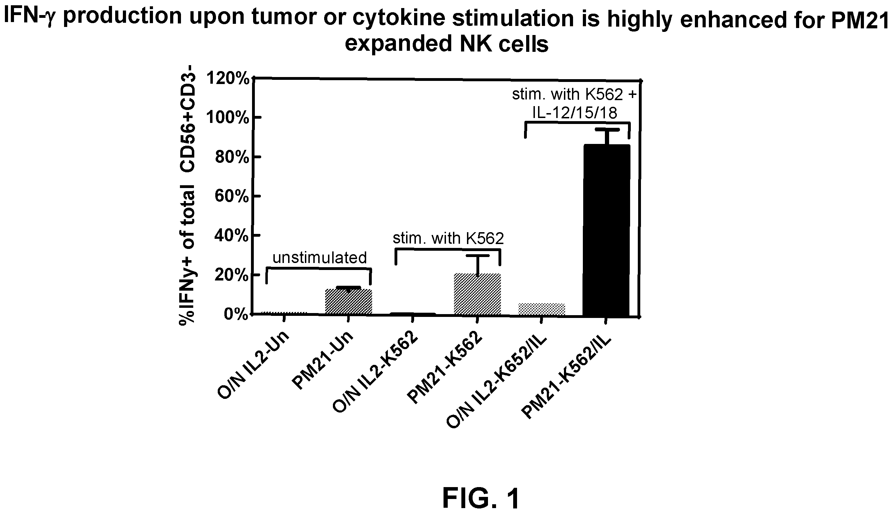

11. Figure 1 shows that PM21-particle expanded NK cells produce higher levels of IFNγ than IL-2 activated NK cells. NK cells were expanded with PM21-particles for 12 days or preactivated with 100 U IL-2 overnight and then IFNγ production was analyzed in unstimulated NK cells (left) or in cells that were exposed to K562 cells alone (center) or together with cytokines (IL-12, IL-15 and IL-18) (right). Unstimulated and stimulated NK cells were permeabilized and stained with anti-IFNγ Ab and analyzed by flow cytometry.

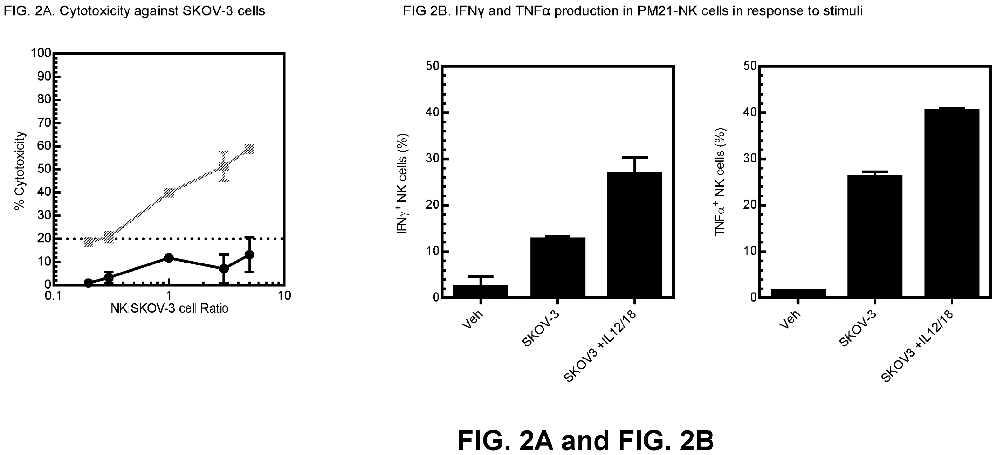

12. Figure 2A and 2B shows that PM21 NK cells are cytotoxic against SKOV-3 cells and secrete IFNγ and TNFα upon stimulation. Figure 2A shows that NK cells were expanded with PM21-particles for 14 days or activated with IL2 (100 U, O/N) and then were added to SKOV-3 cells at increasing ratios. Cells were incubated for 30 minutes and the viability of GFP+ SKOV-3 cells was determined using Annexin V-v450 staining while cytotoxicity was determined based on reference well with SKOV-3 only cells. Figure 2B shows expanded PM21-NK cells were left unstimulated or were exposed to SKOV-3 cells alone or in presence of cytokines IL12/18 for 4 hours. Cells were stained for surface markers, permebialized and stained for IFNγ and TNFα and then analyzed by flow cytometry.

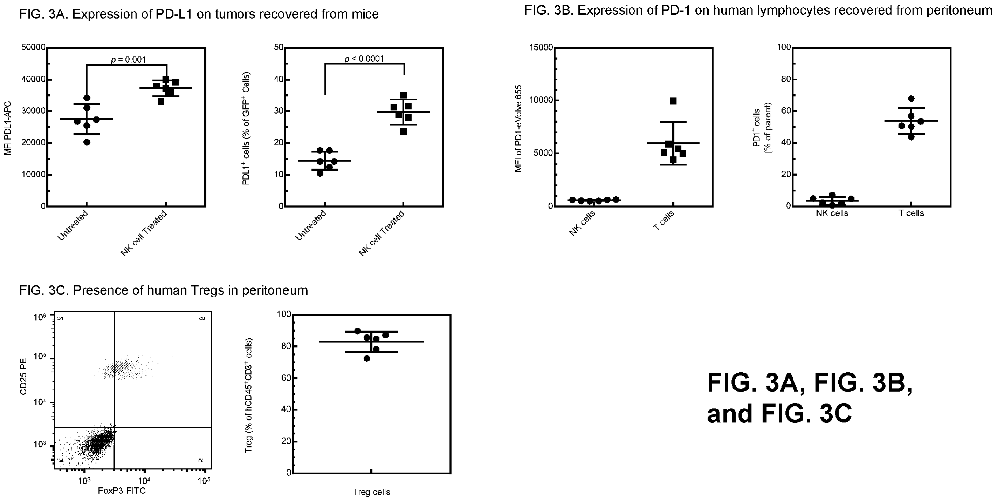

13. Figures 3A, 3B, and 3C show that adoptively transferred PM21 NK cells induce PD-L1 on SKOV-3 cells and drive Treg expansion *in vivo.* NSG mice were implanted with 1×10⁶ SKOV-3 cells *i.p..* Mice were treated with 10×10⁶ of PM21 NK cells or with vehicle control on day 8 and 13 followed by IL2 injections (25000 U, 3x/week). Mice were sacrificed on day 12 post 1^{st} NK cell injection, tumors and peritoneal wash were collected for analysis. Figure 3A shows tumors were perfused to obtain single cell suspension and were stained with anti-PD-L1. Figure 3B shows peritoneal wash cells were analyzed for presence of PD-1 expression on NK cells and T cells as well as for presence of regulatory T cells (CD3+CD4+CD25 ^{Bright} FoxP⁺)(Figure 3C).

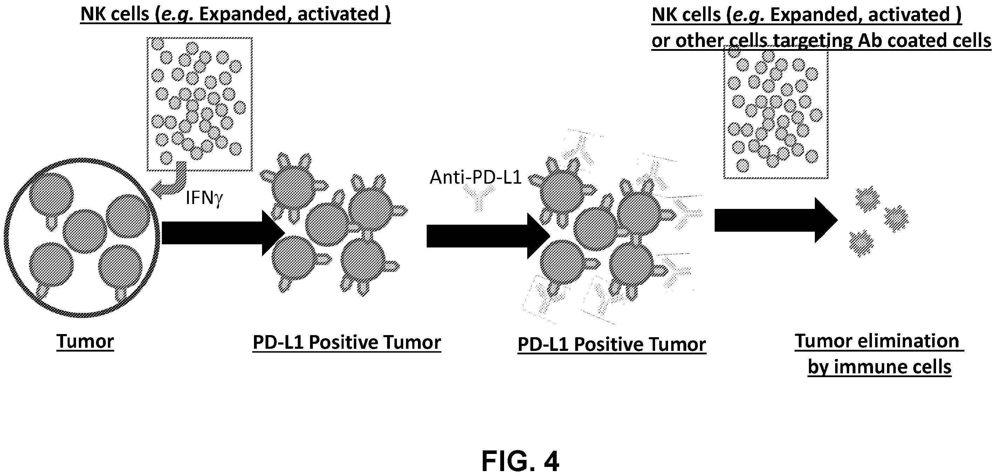

14. Figure 4 shows a schematic for IFNγ secreting NK cells to induce PD-L1 expression and combination treatment with anti-PDL1 mAb. PD-L1 negative (or low) tumors are challenged with adoptively transferred NK cells. NK cells respond to tumor by secretion of high levels of IFN-γ which in turns leads to induction of high levels of PD-L1 on tumor cells (defense mechanism that leads to immune suppression). PD-L1 can now be highly efficiently targeted with anti-PD-L1 antibody in combination with adoptive transfer of cells capable of antibody dependent cell cytotoxicity (ADCC) such as CD16⁺ NK cells. Induction of PD-L1 on tumors will also provide an universal, targetable ligand for tumor treatment with NK cells by ADCC or CARs-T cells targeted against this molecule for universal platform for cancer treatment.

### IV. DETAILED DESCRIPTION

### A. Definitions

15. As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

16. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

17. In this specification and in the claims which follow, reference will be made to a number of terms which shall be defined to have the following meanings:

18. "Optional" or "optionally" means that the subsequently described event or circumstance may or may not occur, and that the description includes instances where said event or circumstance occurs and instances where it does not.

### B. Cancer therapies

19. As explained previously, expression of PD-L1 on most tumors is induced in response to IFNγ secreted by CD8 T cells recruited to the tumor site. This induction of PD-L1 expression on tumor initiates a cascade of events that ultimately creates an immunosuppressive environment and also directly blocks the function of cytotoxic T cells through interaction with PD-1 receptor and leads to the anergy and apoptosis of cytotoxic T cells. These changes then aid tumor progression and metastasis. The new anti-PD-L1/anti-PD-1 therapies were able to unleash the paralyzed immune system in cancers where the initial immune response to tumor was halted by the induction of PD-L1 and the subsequent immunosuppressive cascade. However, as previously noted, the anti-PDL1/PD1 therapies are limited to cancers where PDL1 is already induced.

20. The present disclosure seeks to treat cancers and improve upon anti-PDL1 therapy through the purposeful induction of the immunosuppressive PDL1 on a cancer followed by the use of anti-PDL1 therapy. Specifically, disclosed herein is a counterintuitive treatment that deliberately induces PDL1 expression on cancerous tissue and then obliterates the cells expressing PDL1 with an anti-PDL1 antibody. This deliberate induction occurs through the use of NK cells activated by contact with one or more of PM21 particles, EX21 exosomes and FC21 feeder cells. The adoptive NK cells can be of various sources (such as, for example, NK cells from peripheral blood, umbilical cord blood, placental blood, from a CMV seropositive donor, or derived from other sources such as induced pluripotent stem cells, embryonic stem cells, or immortalized NK cell like cell lines such, as NK92) and are treated by activation or expansion methods that including contact with one or more of PM21 particles, EX21 exosomes and FC21 feeder cells. For example, the PM21 particles, EX21 exosomes, FC21 feeder cells activated and/or expanded adoptive NK cells can be XM NK cells.

21. Human NK cells are a subset of peripheral blood lymphocytes defined by the expression of CD56 or CD 16 and the absence of T cell receptor (CD3). NK cells sense and kill target cells that lack major histocompatibility complex (MHC)-class I molecules. NK cell activating receptors include, among others, the natural cytotoxicity receptors (NKp30, NKp44 and NKp46), and lectin-like receptors NKG2D and DNAM-1. Their ligands are expressed on stressed, transformed, or infected cells but not on normal cells, making normal cells resistant to NK cell killing. NK cell activation is negatively regulated via inhibitory receptors, such as killer immunoglobin (Ig)-like receptors (KIRs), NKG2A /CD94, and leukocyte Ig-like receptor- 1 (LIR-1). Engagement of one inhibitory receptor may be sufficient to prevent target lysis. Hence NK cells efficiently target cells that express many stress-induced ligands, and few MHC class I ligands.

22. NK cells efficiently destroy tumor cells, stressed cells, and virally infected cells by a variety of different methods. The first is by directly engaging target cells, permeating their membranes, and then injecting a protein that cleaves and activates several apoptotic proteins, thereby initiating programmed cell death (apoptosis) of the targeted cell. The surface of an NK cell also contains protein ligands that can bind and activate receptors, such as the receptor for tumor-necrosis factor (TNF)-related apoptosis-inducing ligand (TRAIL), on target cells that turn on internal signals for apoptotic programmed cell death.

23. NK cells also secrete IFN-γ when activated by a malignant or virally compromised cell. IFN-γ is an important activator of macrophages and inducer of Class II major histocompatibility complex (MHC) molecule expression. However, as noted previously, IFN-γ also has the negative effect of inducing tumor cells to express PDL1 which has immunosuppressive properties. Nevertheless, by using NK cells to target tumor and express IFN-γ in the tumor environment, as opposed to systemic IFN-γ that would be toxic, cancer cells can be forced to induce PDL1 and an anti-PDL1 antibody can then be used to obliterate the tumor and block the immunosuppressive effects of PDL1. Effectively, the use of NK cells overcomes the limitations of anti-PDL1 antibody therapy alone.

24. Nevertheless, the efficacy of NK cell immunotherapy is dependent on the dose of NK cells administered to the patient or reached after infusion through *in vivo* expansion. Currently available techniques are limited by their inability to achieve the level of NK cell expansion required to achieve a therapeutic effect in a patient. The lack of a clinical activation or expansion protocol is a major barrier to the progress of NK cell-based immunotherapy. Current *ex vivo* activation and expansion protocols use a combination of high dose cytokines with activating ligands expressed on leukemia-derived feeder/stimulator cell lines, posing a significant downside for transfer to clinical settings in most centers and are not amenable for direct *in vivo* expansion or activation.

25. To be effective as a cancer treatment method, it is desirable to achieve a degree of NK cell activation and/or expansion that reaches an effective therapeutic dose. NK cells proliferate in an *in vitro* culture exponentially and preferentially within a mixture of peripheral blood mononuclear cells (PBMC) when stimulated cytokines (such as IL-15 or IL-21) and ligands for activating receptors (such as 4-1BBL) are expressed on the surface of stimulator cells. For cytokines IL-15 and IL-21, cross-presentation of membrane bound interleukin, as in normal dendritic cells, induces expansion of NK cells more potently than the soluble form of these cytokines. Moreover, under such stimulation conditions, only a low concentration of soluble IL-2 is required for NK cell survival, thus allowing for selective expansion of NK cells within a PBMC mixture without observable proliferation of T cells. The soluble form of IL-15 and IL-21 cytokines or high dose IL-2 stimulate more potently the proliferation of T cells than of NK cells.

26. By contrast, stimulation with membrane bound IL-21, for example on the surface of PM21 particles, EX21 exosomes, or FC21 feeder cells, was found to stimulate continuous propagation of NK cells over countless generations allowing for continuous expansion of NK cells provided that the culture is periodically replenished with fresh stimulatory cells. While these methods allow for efficient in vitro NK cell expansion, the need for live feeder cells makes the methodology difficult to transfer to clinical settings that do not have large GMP facility and capability. Also, NK cells that are infused into the patient will likely stop dividing due to the lack of continued stimulation by the feeders. Furthermore, there is still a lack of information about the ability of in vitro cultured NK cells to function as intended when re-infused into a patient. Through the use of plasma membrane (PM) particles, exosomes (EX), or feeder cells (FC) comprising one or more activating agents, stimulatory peptides, cytokines, and/or adhesion molecules to contact and activate and/or expand NK cells these hurdles are overcome. Examples of NK cell activating agents and stimulatory peptides include 41BBL, IL-2, IL-12, IL-21, IL-18, MICA, LFA-1, 2B4, BCM/SLAMF2, CCR7 and/or other homing receptors. Examples of cytokines include IL-2, IL-12, IL-21, and IL-18. Examples of adhesion molecules include LFA-1, MICA, BCM/SLAMF2. For example, a plasma membrane particle (PM particle), Feeder cells (FC) or or exosomes (EX) prepared from feeder cells expressing membrane bound IL-21 (PM21 particles, FC21 cells, and EX21 exosomes, respectively). The membrane bound IL-21 expressing FC21 cells, PM21 particles, and EX21 exosomes can further comprise additional one or more activating agents, stimulatory peptides, cytokines, and/or adhesion molecules including, but not limited to 41BBL, IL-2, IL-12, IL-18, MICA, LFA-1, 2B4, BCM/SLAMF2, CCR7 (for example, PM21 particle, EX21 exosome, or FC cell expressing 41BBL and membrane bound interleukin-21). Accordingly, in one aspect, disclosed herein is an anti-PDL1 antibody and a component comprising activated NK cells for use in treating a cancer in a subject, wherein the activated NK cells (such as, for example, XM NK cells, NK92 cells, or any NK cell having been stimulated by IL-21) are obtained by contacting a naive NK cell population with PM21 particles, EX21 exosomes, or FC21 feeder cells.

27. It is understood and herein contemplated that PM21 particles, EX21 exosomes, and/or FC21 feeder cells can not only to activate and/or expand exogenous NK cell populations (such as, for example, XM NK cells, NK92 cells, or any NK cell having been stimulated by membrane bound IL-21 ), but can activate and/or expand endogenous NK cell populations (such as, for example, XM NK cells, NK92 cells, or any NK cell having been stimulated by membrane bound IL-21 surface expressing NK cells). Moreover, the PM21 particles, EX21 exosomes, and/or PC21 feeder cells can have similar functional properties to the activated and/or expanded NK cells.

28. The plasma membrane particle or exosome can be purified from NK cell stimulating feeder cells. NK cell stimulating feeder cells for use in making the plasma membrane particles (for example PM21 particles) and exosomes (for example FC21 exosomes) disclosed herein can be either irradiated autologous or allogeneic peripheral blood mononuclear cells (PBMCs) or nonirradiated autologous or PBMCs, RPMI8866, NK-92, NK-92MI, NK-YTS, NK, NKL, KIL, KIL C.2, NK 3.3, NK-YS, HFWT, K562 cells, NK cells transfected with membrane bound IL-15 and 41BBL (such as, for example, NK-92, NK-92MI, NK-YTS, NK, NKL, KIL, KIL C.2, NK 3.3, NK-YS, HFWT, and/or K562 cells transfected with membrane bound IL-21), NK cells transfected with membrane bound IL-21 (such as, for example, NK-92, NK-92MI, NK-YTS, NK, NKL, KIL, KIL C.2, NK 3.3, NK-YS, HFWT, and/or K562 cells transfected with membrane bound IL-15 and 41BBL), NK cells transfected with membrane bound IL-21 and 41BBL (such as, for example, NK-92, NK-92MI, NK-YTS, NK, NKL, KIL, KIL C.2, NK 3.3, NK-YS, HFWT, and/or K562 cells transfected with membrane bound IL-21 and 41BBL), or EBV-LCL. In some aspects, the NK cell feeder cells can be NK-92, NK-92MI, NK-YTS, NK, NKL, KIL, KIL C.2, NK 3.3, NK-YS, HFWT, and/or K562 cells transfected with membrane bound IL-21 and 41BBL or NK-92, NK-92MI, NK-YTS, NK, NKL, KIL, KIL C.2, NK 3.3, NK-YS, HFWT, and/or K562 cells transfected with membrane bound IL-15 and 41BBL.

29. It is understood and herein contemplated that the activation and/or expansion of NK cells occurs ex vivo prior to administration of NK cells (such as, for example, XM NK cells, NK92 cells, or any NK cell having been stimulated by membrane bound IL-21, or any types of NK cell derived from various sources or activation methods). For example, the NK cells (such as, for example, XM NK cells, NK92 cells, or any NK cell having been stimulated by membrane bound IL-21) can be expanded and/or activated ex vivo prior to administration by contacting NK cells (such as, for example, XM NK cells, NK92 cells, or any NK cell having been stimulated by membrane bound IL-21) with PM21 particles, EX21 exosomes, or FC21 cells for between 1 and 28 days prior and more preferably 1 and 21 days prior to administration of the XM NK cells. For example the NK cells (such as, for example, XM NK cells, NK92 cells, or any NK cell having been stimulated by membrane bound IL-21) can be contacted with PM21 particles, EX21 exosomes, or FC21 cells for at least 1, 2, 3, 4, 5, 6, 7, 8 9, 10, 11, 12, 13, 14, 15, 16, 17, 18, 19, 20, 21, 22, 23, 24, 25, 26, 27, or 28 days prior to administration of the NK cells (such as, for example, XM NK cells, NK92 cells, or any NK cell having been stimulated by membrane bound IL-21).

30. The benefit of the activation and/or expansion of the NK cells (such as, for example, XM NK cells, NK92 cells, or any NK cell having been stimulated by membrane bound IL-21) does not stop with the administration of the NK cells (such as, for example, XM NK cells, NK92 cells, or any NK cell having been stimulated by membrane bound IL-21) to the subject. Continued and further expansion and/or activation of NK cells (such as, for example, XM NK cells, NK92 cells, or any NK cell having been stimulated by membrane bound IL-21) can occur *in vivo* through direct administration of PM21 particles and/or EX21 exosomes to the subject. Thus, in one aspect, the subject NK cells (such as, for example, XM NK cells, NK92 cells, or any NK cell having been stimulated by membrane bound IL-21) and an anti-PDL1 antibody for use as described herein are for administration with PM21 particles and/or EX21 exosomes. The administration of PM21 particles and/or EX21 exosomes can occur currently with and/or following administration of the NK cells (such as, for example, XM NK cells, NK92 cells, or any NK cell having been stimulated by membrane bound IL-21, or any types of NK cell derived from various sources or activation methods) to the subject. For example, PM21 particles and/or EX21 exosomes can be administered at least 1, 2, 3, 4, 5, 6, or 7 times per week following the administration of the NK cells (such as, for example, XM NK cells, or any IL-21 surface expressing NK cells).

31. It is further recognized that additional benefit to the NK cells (such as, for example, XM NK cells, or any IL-21 surface expressing NK cells) can occur through exposure of the NK cells (such as, for example, XM NK cells, NK92 cells, or any NK cell having been stimulated by membrane bound IL-21, or any types of NK cell derived from various sources or activation methods) to one or more of IL-12, IL-15, and/or IL-18. This exposure can occur prior to administration of the NK cells (such as, for example, XM NK cells, NK92 cells, or any NK cell having been stimulated by membrane bound IL-21, or any types of NK cell derived from various sources or activation methods) to the subject, concurrently with, and/or after administration of the NK cells to the subject. In one aspect, the one or more of IL-12, IL-15, and/or IL-18 can be administered to the NK cells (such as, for example, XM NK cells, NK92 cells, or any NK cell having been stimulated by membrane bound IL-21, or any types of NK cell derived from various sources or activation methods) during the *ex vivo* culture of the NK cells. Added benefit of IL-12, IL-15, and IL-18 can also be obtained via administration of IL-15, IL-12, and IL-18 directly to the subject prior to, concurrently with, and even continuing after the administration of the NK cells to the subject. In one aspect, disclosed herein are NK cells (such as, for example, XM NK cells, or any NK cell having been stimulated by membrane bound IL-21, or any types of NK cell derived from various sources or activation methods) and an anti-PDL1 antibody for use as defined herein for administration with one or more of IL-12, IL-15, and/or IL-18, wherein the one or more of IL-12, IL-15, and/or IL-18 is for administration prior to, concurrently with, and/or following administration of the NK cells (such as, for example, XM NK cells, or any NK cell having been stimulated by membrane bound IL-21, or any types of NK cell derived from various sources or activation methods) to the subject.

32. As noted previously, PDL1 is not necessarily present in all cancers and needs to be present for anti-PDL1 therapy to be effective. Thus, for an anti-PDL1 antibody to be effective in a cancer therapy, PDL1 needs to be induced which is accomplished in the present invention through the administration of memory-like NK cells, or PM21, or FC21 NK cells, or any types of NK cell derived from various sources or activation methods which express high amounts of IFN-γ in response to sensing a tumor and thereby induce PDL1 expression on the tumor. In one aspect, the NK cells (such as, for example, XM NK cells, or NK cell having been stimulated by membrane bound IL-21, or any types of NK cell derived from various sources or activation methods) can be administered concurrently with the anti-PDL1 antibody. However, because antibodies can have a half-life shorter, it is contemplated that the NK cells that would secrete IFNγ in response to a tumor can also be administered prior to administration of the anti-PDL1 antibody. For example, it is contemplated herein that the NK cells can be administered between 1 and 21 days and more preferably 1 and 14 days prior to administration of the anti-PDL1 antibody. For example, disclosed herein are NK cells and an anti-PDL1 antibody for use as described herein wherein the NK cells are administered 1, 2, 3, 4, 5, 6, 7, 8 9, 10, 11, 12, 13, 14, 15, 16, 17, 18, 19, 20, or 21 prior to administration of the anti-PDL1 antibody.

33. The source of NK cells can be critical to the efficacy of the therapy as rejection of transferred NK cells would thwart the therapeutic process. Accordingly, it is contemplated herein that the NK cells (such as, for example, XM NK cells, or NK cell having been stimulated by membrane bound IL-21, or any types of NK cell derived from various sources or activation methods) were derived from an autologous, haploidentical, or allogeneic donor source of NK cells. The NK cells may also be derived umbilical cord blood, placental blood, or from various types of stem cells, or from NK cell like cell lines such as NK92.

34. Activating and/or expanding endogenous NK cells via directly administering to the subject PM21 particles and/or EX21 exosomes can also avoid completely any potential for rejection of transferred NK cells. Administration can be made as often as needed to achieve the desired amount of XM NK cells in the subject. For example, PM21 particles and/or EX21 exosomes can be administered at least 1, 2, 3, 4, 5, 6, or 7 times per week following the administration of the initial administration of the PM21 particles and/or EX21 exosomes.

35. As noted above, PDL1 is not necessarily present in all cancers and needs to be present for anti-PDL1 antibody therapy to be effective and PDL1 needs to be induced. Thus, administration of PM21 particles and EX21 exosomes can expand and/or activate endogenous NK cells to transform into XM NK cells in vivo and enhance secretion IFN-y to the tumor which induces PDL1 expression and allows for effective use of anti-PDL1 antibodies. In one aspect, PM21 particles and/or EX21 exosomes can be administered concurrently with the anti-PDL1 antibody. However, because antibodies can have a half-life shorter, it is contemplated that it can be advantageous to expand and/or activate endogenous XM NK cells prior to administration of the anti-PDL1 antibody. For example, it is contemplated herein that the PM21 particles and/or EX21 exosomes can be administered between 1 and 21 days and more preferably 1 and 14 days prior to administration of the anti-PDL1 antibody. For example, the PM21 particles and/or EX21 exosomes may be administered 1, 2, 3, 4, 5, 6, 7, 8 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 days prior to administration of the anti-PDL1 antibody.

36. As noted above, added benefit to the XM NK cells can occur through the additional exposure to IL-12, IL-15, and IL-18. This benefit can also be obtained via administration of IL-15, IL-12, and IL-18 directly to the subject prior to, concurrently with, and even continuing after the administration of PM21 particles and/or EX21 exosomes to the subject. Where XM NK cells that are expanded and/or activated by PM21 particles, EX21 exosomes, or FC21 feeder cells are further exposed to cytokines, the resulting XM NK cells can be referred to as CaPM21 NK cells (cytokine and PM21 particle stimulated XM NK cells), CEX21 NK cells (cytokine and EX21 exosome stimulated XM NK cells), and CFC21 NK cells (cytokine and FC21 feeder cell stimulated XM NK cells), respectively.

37. A representative but non-limiting list of cancers that the disclosed compositions can be used to treat is the following: lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, or pancreatic cancer.

38. It is understood and herein contemplated that there are a host of other diseases and conditions other than cancers that can be treated by the uses disclosed herein. Accordingly, also disclosed herein are a component comprising activated NK cells and an anti-PDL1 antibody for use in treating an infection in a subject (e.g. a viral, bacterial, fungal, or parasitic infection), wherein the activated NK cells are obtained by contacting a naive NK cell population with at least one or more of PM21 particles, EX21 exosomes and FC21 feeder cells.

39. The viral infection may be from a virus selected from the group of viruses consisting of Herpes Simplex virus- 1, Herpes Simplex virus-2, Varicella-Zoster virus, Epstein-Barr virus, Cytomegalovirus, Human Herpes virus-6, Variola virus, Vesicular stomatitis virus, Hepatitis A virus, Hepatitis B virus, Hepatitis C virus, Hepatitis D virus, Hepatitis E virus, Rhinovirus, Coronavirus, Influenza virus A, Influenza virus B, Measles virus, Polyomavirus, Human Papilomavirus, Respiratory syncytial virus, Adenovirus, Coxsackie virus, Dengue virus, Mumps virus, Poliovirus, Rabies virus, Rous sarcoma virus, Reovirus, Yellow fever virus, Ebola virus, Marburg virus, Lassa fever virus, Eastern Equine Encephalitis virus, Japanese Encephalitis virus, St. Louis Encephalitis virus, Murray Valley fever virus, West Nile virus, Rift Valley fever virus, Rotavirus A, Rotavirus B, Rotavirus C, Sindbis virus, Simian Immunodeficiency virus, Human T-cell Leukemia virus type-1, Hantavirus, Rubella virus, Simian Immunodeficiency virus, Human Immunodeficiency virus type-1, and Human Immunodeficiency virus type-2.

40. The bacterial infection may be from bacteria selected from the group of bacteria consisting of M. tuberculosis, M. bovis, M. bovis strain BCG, BCG substrains, M. avium, M. intracellular, M. africanum, M. kansasii, M. marinum, M. ulcerans, M. avium subspecies paratuberculosis, Nocardia asteroides, other Nocardia species, Legionella pneumophila, other Legionella species, Salmonella typhi, other Salmonella species, Shigella species, Yersinia pestis, Pasteurella haemolytica, Pasteurella multocida, other Pasteurella species, Actinobacillus pleuropneumoniae, Listeria monocytogenes, Listeria ivanovii, Brucella abortus, other Brucella species, Cowdria ruminantium, Chlamydia pneumoniae, Chlamydia trachomatis, Chlamydia psittaci, Coxiella burnetii, other Rickettsial species, Ehrlichia species, Staphylococcus aureus, Staphylococcus epidermidis, Streptococcus pneumoniae, Streptococcus pyogenes, Streptococcus agalactiae, Bacillus anthracis, Escherichia coli, Vibrio cholerae, Campylobacter species, Neiserria meningitidis, Neiserria gonorrhea, Pseudomonas aeruginosa, other Pseudomonas species, Haemophilus influenzae, Haemophilus ducreyi, other Hemophilus species, Clostridium tetani, other Clostridium species, Yersinia enterolitica, and other Yersinia species.

41. The fungal infection may be from fungi selected from the group of fungi consisting of Candida albicans, Cryptococcus neoformans, Histoplama capsulatum, Aspergillus fumigatus, Coccidiodes immitis, Paracoccidiodes brasiliensis, Blastomyces dermitidis, Pneumocystis carnii, Penicillium marneffi, and Alternaria alternata.

42. The parasitic infection may be from a parasite selected from the group of parasitic organisms consisting of Toxoplasma gondii, Plasmodium falciparum, Plasmodium vivax, Plasmodium malariae, other Plasmodium species, Trypanosoma brucei, Trypanosoma cruzi, Leishmania major, other Leishmania species, Schistosoma mansoni, other Schistosoma species, and Entamoeba histolytica.

### C. Compositions

43. Disclosed are the components to be used to prepare the disclosed compositions as well as the compositions themselves for use as described herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular anti-PDL1 antibody, PM21 particle, EX21 exosome, or FC21 feeder cell is disclosed and discussed and a number of modifications that can be made to a number of molecules including the anti-PDL1 antibody, PM21 particle, EX21 exosome, or FC21 feeder cell are discussed, specifically contemplated is each and every combination and permutation of anti-PDL1 antibody, PM21 particle, EX21 exosome, or FC21 feeder cell and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

44. The disclosed cancer therapies utilize anti-PDL1 antibodies in combination with PM21, EX21, or FC21 XM NK cells and optionally PM21 particles and/or EX21 exosomes. Thus, in one aspect, the anti-cancer therapies comprise PM21, EX21, or FC21 XM NK cells and an anti-PDL1 antibody. Accordingly, in one aspect are anti-cancer therapies comprising PM21, EX21, or FC21 XM NK cells and an anti-PDL1 antibody further comprising PM21 particles and/or EX21 exosomes. Additionally, any of the above disclosed anti-cancer therapies can comprise one or more of IL-12, IL-15, and IL-18.

### 1. Antibodies

45. The anti-cancer therapies disclosed herein comprise the use of an anti-PDL1 antibody. It is understood and herein contemplated that the disclosed therapies can comprise and/or utilize any known anti-PDL1 antibody, and in particular, any previously shown to have a therapeutic effect including, but not limited to atezolizumab by Genentech.

### (1) Antibodies Generally

46. The term "antibodies" is used herein in a broad sense and includes both polyclonal and monoclonal antibodies. In addition to intact immunoglobulin molecules, also included in the term "antibodies" are fragments or polymers of those immunoglobulin molecules, and human or humanized versions of immunoglobulin molecules or fragments thereof, as long as they are chosen for their ability to interact with PDL1 such that PDL1 is inhibited from interacting with PD1. The antibodies can be tested for their desired activity using the *in vitro* assays described herein, or by analogous methods, after which their *in vivo* therapeutic and/or prophylactic activities are tested according to known clinical testing methods. There are five major classes of human immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. One skilled in the art would recognize the comparable classes for mouse. The heavy chain constant domains that correspond to the different classes of immunoglobulins are called alpha, delta, epsilon, gamma, and mu, respectively.

47. The term "monoclonal antibody" as used herein refers to an antibody obtained from a substantially homogeneous population of antibodies, i.e., the individual antibodies within the population are identical except for possible naturally occurring mutations that may be present in a small subset of the antibody molecules. The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, as long as they exhibit the desired antagonistic activity.

48. The disclosed monoclonal antibodies can be made using any procedure which produces mono clonal antibodies. For example, disclosed monoclonal antibodies can be prepared using hybridoma methods, such as those described by Kohler and Milstein, Nature, 256:495 (1975). In **a** hybridoma method, a mouse or other appropriate host animal is typically immunized with an immunizing agent to elicit lymphocytes that produce or are capable of producing antibodies that will specifically bind to the immunizing agent. Alternatively, the lymphocytes may be immunized *in vitro.*

49. The monoclonal antibodies may also be made by recombinant DNA methods. DNA encoding the disclosed monoclonal antibodies can be readily isolated and sequenced using conventional procedures (e.g., by using oligonucleotide probes that are capable of binding specifically to genes encoding the heavy and light chains of murine antibodies). Libraries of antibodies or active antibody fragments can also be generated and screened using phage display techniques, e.g., as described in U.S. Patent No. 5,804,440 to Burton et al. and U.S. Patent No. 6,096,441 to Barbas et al.

*50. In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published Dec. 22, 1994 and U.S. Pat. No. 4,342,566. Papain digestion of antibodies typically produces two identical antigen binding fragments, called Fab fragments, each with a single antigen binding site, and a residual Fc fragment. Pepsin treatment yields a fragment that has two antigen combining sites and is still capable of cross-linking antigen.

51. As used herein, the term "antibody or fragments thereof" encompasses chimeric antibodies and hybrid antibodies, with dual or multiple antigen or epitope specificities, and fragments, such as F(ab')2, Fab', Fab, Fv, sFv, and the like, including hybrid fragments. Thus, fragments of the antibodies that retain the ability to bind their specific antigens are provided. For example, fragments of antibodies which maintain PDL1 binding activity are included within the meaning of the term "antibody or fragment thereof." Such antibodies and fragments can be made by techniques known in the art and can be screened for specificity and activity according to the methods set forth in the Examples and in general methods for producing antibodies and screening antibodies for specificity and activity (See Harlow and Lane. Antibodies, A Laboratory Manual. Cold Spring Harbor Publications, New York, (1988)).

52. Also included within the meaning of "antibody or fragments thereof" are conjugates of antibody fragments and antigen binding proteins (single chain antibodies).

53. The fragments, whether attached to other sequences or not, can also include insertions, deletions, substitutions, or other selected modifications of particular regions or specific amino acids residues, provided the activity of the antibody or antibody fragment is not significantly altered or impaired compared to the non-modified antibody or antibody fragment. These modifications can provide for some additional property, such as to remove/add amino acids capable of disulfide bonding, to increase its bio-longevity, to alter its secretory characteristics, etc. In any case, the antibody or antibody fragment must possess a bioactive property, such as specific binding to its cognate antigen. Functional or active regions of the antibody or antibody fragment may be identified by mutagenesis of a specific region of the protein, followed by expression and testing of the expressed polypeptide. Such methods are readily apparent to a skilled practitioner in the art and can include site-specific mutagenesis of the nucleic acid encoding the antibody or antibody fragment. (Zoller, M.J. Curr. Opin. Biotechnol. 3:348-354, 1992).

54. As used herein, the term "antibody" or "antibodies" can also refer to a human antibody and/or a humanized antibody. Many non-human antibodies (e.g., those derived from mice, rats, or rabbits) are naturally antigenic in humans, and thus can give rise to undesirable immune responses when administered to humans. Therefore, the use of human or humanized antibodies in the methods serves to lessen the chance that an antibody administered to a human will evoke an undesirable immune response.

### (2) Human antibodies

55. The disclosed human antibodies can be prepared using any technique. The disclosed human antibodies can also be obtained from transgenic animals. For example, transgenic, mutant mice that are capable of producing a full repertoire of human antibodies, in response to immunization, have been described (see, e.g., Jakobovits et al., Proc. Natl. Acad. Sci. USA, 90:2551-255 (1993); Jakobovits et al., Nature, 362:255-258 (1993); Bruggermann et al., Year in Immunol., 7:33 (1993)). Specifically, the homozygous deletion of the antibody heavy chain joining region (J(*H*)) gene in these chimeric and germ-line mutant mice results in complete inhibition of endogenous antibody production, and the successful transfer of the human germ-line antibody gene array into such germ-line mutant mice results in the production of human antibodies upon antigen challenge. Antibodies having the desired activity are selected using Env-CD4-co-receptor complexes as described herein.

### (3) Humanized antibodies

56. Antibody humanization techniques generally involve the use of recombinant DNA technology to manipulate the DNA sequence encoding one or more polypeptide chains of an antibody molecule. Accordingly, a humanized form of a non-human antibody (or a fragment thereof) is a chimeric antibody or antibody chain (or a fragment thereof, such as an sFv, Fv, Fab, Fab', F(ab')2, or other antigen-binding portion of an antibody) which contains a portion of an antigen binding site from a non-human (donor) antibody integrated into the framework of a human (recipient) antibody.

57. To generate a humanized antibody, residues from one or more complementarity determining regions (CDRs) of a recipient (human) antibody molecule are replaced by residues from one or more CDRs of a donor (non-human) antibody molecule that is known to have desired antigen binding characteristics (e.g., a certain level of specificity and affinity for the target antigen). In some instances, Fv framework (FR) residues of the human antibody are replaced by corresponding non-human residues. Humanized antibodies may also contain residues which are found neither in the recipient antibody nor in the imported CDR or framework sequences. Generally, a humanized antibody has one or more amino acid residues introduced into it from a source which is non-human. In practice, humanized antibodies are typically human antibodies in which some CDR residues and possibly some FR residues are substituted by residues from analogous sites in rodent antibodies. Humanized antibodies generally contain at least a portion of an antibody constant region (Fc), typically that of a human antibody (Jones et al., Nature, 321:522-525 (1986), Reichmann et al., Nature, 332:323-327 (1988), and Presta, Curr. Opin. Struct. Biol., 2:593-596 (1992)).

58. Methods for humanizing non-human antibodies are well known in the art. For example, humanized antibodies can be generated according to the methods of Winter and co-workers (Jones et al., Nature, 321:522-525 (1986), Riechmann et al., Nature, 332:323-327 (1988), Verhoeyen et al., Science, 239:1534-1536 (1988)), by substituting rodent CDRs or CDR sequences for the corresponding sequences of a human antibody. Methods that can be used to produce humanized antibodies are also described in U.S. Patent No. 4,816,567 (Cabilly et al.), U.S. Patent No. 5,565,332 (Hoogenboom et al.), U.S. Patent No. 5,721,367 (Kay et al.), U.S. Patent No. 5,837,243 (Deo et al.), U.S. Patent No. 5, 939,598 (Kucherlapati et al.), U.S. Patent No. 6,130,364 (Jakobovits et al.), and U.S. Patent No. 6,180,377 (Morgan et al.).

### (4) Administration of antibodies

59. Administration of the antibodies can be done as disclosed herein. Nucleic acid approaches for antibody delivery also exist. The broadly neutralizing anti-PDL1 antibodies and antibody fragments can also be administered to patients or subjects as a nucleic acid preparation (e.g., DNA or RNA) that encodes the antibody or antibody fragment, such that the patient's or subject's own cells take up the nucleic acid and produce and secrete the encoded antibody or antibody fragment. The delivery of the nucleic acid can be by any means, as disclosed herein, for example.

### 2. Pharmaceutical carriers/Delivery of pharamceutical products

60. As described above, the compositions can also be administered *in vivo* in a pharmaceutically acceptable carrier. By "pharmaceutically acceptable" is meant a material that is not biologically or otherwise undesirable, i.e., the material may be administered to a subject, along with the nucleic acid or vector, without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be well known to one of skill in the art.

61. The compositions may be administered orally, parenterally (e.g., intravenously), by intramuscular injection, by intraperitoneal injection, transdermally, extracorporeally, topically or the like, including topical intranasal administration or administration by inhalant. As used herein, "topical intranasal administration" means delivery of the compositions into the nose and nasal passages through one or both of the nares and can comprise delivery by a spraying mechanism or droplet mechanism, or through aerosolization of the nucleic acid or vector. Administration of the compositions by inhalant can be through the nose or mouth via delivery by a spraying or droplet mechanism. Delivery can also be directly to any area of the respiratory system (e.g., lungs) via intubation. The exact amount of the compositions required will vary from subject to subject, depending on the species, age, weight and general condition of the subject, the severity of the allergic disorder being treated, the particular nucleic acid or vector used, its mode of administration and the like. Thus, it is not possible to specify an exact amount for every composition. However, an appropriate amount can be determined by one of ordinary skill in the art using only routine experimentation given the teachings herein.

62. Parenteral administration of the composition, if used, is generally characterized by injection. Injectables can be prepared in conventional forms, either as liquid solutions or suspensions, solid forms suitable for solution of suspension in liquid prior to injection, or as emulsions. A more recently revised approach for parenteral administration involves use of a slow release or sustained release system such that a constant dosage is maintained. See, e.g., U.S. Patent No. 3,610,795.

63. The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells *in vivo.* The following references are examples of the use of this technology to target specific proteins to tumor tissue (Hughes et al., Cancer Research, 49:6214-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

### a) Pharmaceutically Acceptable Carriers

64. The compositions, including antibodies, can be used therapeutically in combination with a pharmaceutically acceptable carrier.

65. Suitable carriers and their formulations are described in Remington: The Science and Practice of Pharmacy (19th ed.) ed. A.R. Gennaro, Mack Publishing Company, Easton, PA 1995. Typically, an appropriate amount of a pharmaceutically-acceptable salt is used in the formulation to render the formulation isotonic. Examples of the pharmaceutically-acceptable carrier include, but are not limited to, saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of composition being administered.

66. Pharmaceutical carriers are known to those skilled in the art. These most typically would be standard carriers for administration of drugs to humans, including solutions such as sterile water, saline, and buffered solutions at physiological pH. The compositions can be administered intramuscularly or subcutaneously. Other compounds will be administered according to standard procedures used by those skilled in the art.

67. Pharmaceutical compositions may include carriers, thickeners, diluents, buffers, preservatives, surface active agents and the like in addition to the molecule of choice. Pharmaceutical compositions may also include one or more active ingredients such as antimicrobial agents, antiinflammatory agents, anesthetics, and the like.

68. The pharmaceutical composition may be administered in a number of ways depending on whether local or systemic treatment is desired, and on the area to be treated. Administration may be topically (including ophthalmically, vaginally, rectally, intranasally), orally, by inhalation, or parenterally, for example by intravenous drip, subcutaneous, intraperitoneal or intramuscular injection. The disclosed antibodies can be administered intravenously, intraperitoneally, intramuscularly, subcutaneously, intracavity, or transdermally.

69. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

70. Formulations for topical administration may include ointments, lotions, creams, gels, drops, suppositories, sprays, liquids and powders. Conventional pharmaceutical carriers, aqueous, powder or oily bases, thickeners and the like may be necessary or desirable.

71. Compositions for oral administration include powders or granules, suspensions or solutions in water or non-aqueous media, capsules, sachets, or tablets. Thickeners, flavorings, diluents, emulsifiers, dispersing aids or binders may be desirable.

72. Some of the compositions may potentially be administered as a pharmaceutically acceptable acid- or base- addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mono-, di-, trialkyl and aryl amines and substituted ethanolamines.

### b) Therapeutic Uses

73. Effective dosages and schedules for administering the compositions may be determined empirically, and making such determinations is within the skill in the art. The dosage ranges for the administration of the compositions are those large enough to produce the desired effect in which the symptoms of the disorder are affected. The dosage should not be so large as to cause adverse side effects, such as unwanted cross-reactions, anaphylactic reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any counterindications. Dosage can vary, and can be administered in one or more dose administrations daily, for one or several days. Guidance can be found in the literature for appropriate dosages for given classes of pharmaceutical products. For example, guidance in selecting appropriate doses for antibodies can be found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

74. Following administration of a disclosed composition, such as an antibody, for treating or inhibiting cancer, the efficacy of the therapeutic antibody can be assessed in various ways well known to the skilled practitioner. For instance, one of ordinary skill in the art will understand that a composition, such as an antibody, disclosed herein is efficacious in treating or inhibiting a cancer in a subject by observing that the composition reduces tumor size or the rate of metastasis.

### 3. Combination treatments

75. The disclosed compositions may be for administration in combination with a known therapeutic for the disease or disorder being treated. For example, the cancer therapies disclosed herein may be used in combination with a known cancer therapeutic such as, but not limited to, a chemotherapeutic, immunotherapeutic, radiation therapy or pain therapeutic.

76. There are two distinct types of immunotherapy: passive immunotherapy uses components of the immune system to direct targeted cytotoxic activity against cancer cells, without necessarily initiating an immune response in the patient, while active immunotherapy actively triggers an endogenous immune response. Passive strategies include the use of the monoclonal antibodies (mAbs) produced by B cells in response to a specific antigen. The development of hybridoma technology in the 1970s and the identification of tumor-specific antigens permitted the pharmaceutical development of mAbs that could specifically target tumor cells for destruction by the immune system. Thus far, mAbs have been the biggest success story for immunotherapy; the top three best-selling anticancer drugs in 2012 were mAbs. Among them is rituximab (Rituxan, Genentech), which binds to the CD20 protein that is highly expressed on the surface of B cell malignancies such as non-Hodgkin's lymphoma (NHL). Rituximab is approved by the FDA for the treatment of NHL and chronic lymphocytic leukemia (CLL) in combination with chemotherapy. Another important mAb is trastuzumab (Herceptin; Genentech), which revolutionized the treatment of HER2 (human epidermal growth factor receptor 2)-positive breast cancer by targeting the expression of HER2.

77. Generating optimal "killer" CD8 T cell responses also requires T cell receptor activation plus co-stimulation, which can be provided through ligation of tumor necrosis factor receptor family members, including OX40 (CD134) and 4-1BB (CD137). OX40 is of particular interest as treatment with an activating (agonist) anti-OX40 mAb augments T cell differentiation and cytolytic function leading to enhanced anti-tumor immunity against a variety of tumors.

78. In some embodiments, the disclosed therapy is for use in combination with adoptive cell therapies (ACT), such as Chimeric Antigen Receptors (CAR), T Cell Receptors (TCR), and Tumor Infiltrating Lymphocytes (TIL).

79. The term "tumor infiltrating lymphocyte" or "TIL" refers to white blood cells that have left the bloodstream and migrated into a tumor. Expansion of lymphocytes, including tumor-infiltrating lymphocytes, such as T cells can be accomplished by any of a number of methods as are known in the art. For example, T cells can be rapidly expanded using non-specific T-cell receptor stimulation in the presence of feeder lymphocytes and interleukin-2 (IL-2), IL-7, IL-15, IL-21, or combinations thereof. The non-specific T-cell receptor stimulus can e.g. include around 30 ng/ml of OKT3, a mouse monoclonal anti-CD3 antibody (available from Ortho-McNeil(R), Raritan, N.J. or Miltenyi Biotec, Bergisch Gladbach, Germany). Alternatively, T cells can be rapidly expanded by stimulation of peripheral blood mononuclear cells (PBMC) in vitro with one or more antigens (including antigenic portions thereof, such as epitope(s), or a cell of the cancer, which can be optionally expressed from a vector, such as an human leukocyte antigen A2 (HLA-A2) binding peptide, e.g., approximately 0.3 µM MART-1 :26-35 (27 L) or gp100:209-217 (210M)), in the presence of a T-cell growth factor, such as around 200-400 IU/ml, such as 300 IU/ml IL-2 or IL-15, with IL-2 being preferred. The in vitro-induced T-cells are rapidly expanded by re-stimulation with the same antigen(s) of the cancer pulsed onto HLA- A2-expressing antigen-presenting cells. Alternatively, the T-cells can be re-stimulated with irradiated, autologous lymphocytes or with irradiated HLA-A2+ allogeneic lymphocytes and IL-2, for example.

80. Specific tumor reactivity of the expanded TILs can be tested by any method known in the art, e.g., by measuring cytokine release (e.g., interferon-gamma) following co-culture with tumor cells. The autologous ACT method disclosed herein may comprise enriching cultured TILs for CD8+ T cells prior to rapid expansion of the cells. Following culture of the TILs in IL-2, the T cells are depleted of CD4+ cells and enriched for CD8+ cells using, for example, a CD8 microbead separation (e.g., using a CliniMACS<plus >CD8 microbead system (Miltenyi Biotec)). A T-cell growth factor that promotes the growth and activation of the autologous T cells may be administered to the mammal either concomitantly with the autologous T cells or subsequently to the autologous T cells. The T-cell growth factor can be any suitable growth factor that promotes the growth and activation of the autologous T-cells.

81. Examples of suitable T-cell growth factors include interleukin (IL)-2, IL-7, IL-15, IL-12 and IL-21 , which can be used alone or in various combinations, such as IL-2 and IL-7, IL-2 and IL-15, IL-7 and IL-15, IL-2, IL-7 and IL-15, IL-12 and IL-7, IL- 12 and IL-15, or IL-12 and IL2.

82. Numerous anti-cancer drugs are also available for combination with the present compositions for use as described herein. The following is a non-exhaustive lists of anti-cancer (anti-neoplastic) drugs that can be used in conjunction with irradiation: Acivicin; Aclarubicin; AcodazoleHydrochloride; AcrQnine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflomithine Hydrochloride; Elsamitrucin; Enloplatin; Enpromate; Epipropidine; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; Fluorouracil; Flurocitabine; Fosquidone; Fostriecin Sodium; Gemcitabine; Gemcitabine Hydrochloride; Gold Au 198; Hydroxyurea; Idarubicin Hydrochloride; Ifosfamide; Ilmofosine; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin; Mitosper; Mitotane; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safmgol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride.

83. In some aspects, the cancer therapeutic, anti-PDL1 antibody, PM21 particles and/or EX21 exosomes can be formulated in the same composition. In some aspects, the cancer therapeutic, anti-PDL1 antibody, PM21 particles and/or EX21 exosomes can be formulated in different compositions.

### D. Examples

84. The following examples are put forth so as to provide those of ordinary skill in the art with a complete disclosure and description of how the compositions for use claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

85. New anti-PD-L1/anti-PD-1 therapies are able to unleash the paralyzed immune system in cancers where the initial immune response to tumor was halted by the induction of PD-L1 and the subsequent immunosuppressive cascade. Similar response by tumors to express PD-L1 on their cell surface is observed when adoptive cell therapy is performed using highly cytotoxic NK cells such as e.g. PM21-particle expanded NK cells (PM21-NK). These PM21-NK cells secrete far higher amounts of IFN-γ than non-expanded NK cells or IL-2 activated NK cells, in response to tumor targets and cytokines (IL-12, IL-18) that are normally present in the tumor bed (Figure 1). NK cells are also selective at targeting only malignant cell and not healthy cells. Thus, adoptively transferred PM21-NK cells can deliver IFNγ specifically to tumor cells and induce PD-L1 without systemic IFN-γ administration, sparing healthy normal cells. As shown in Figure 2, PM21 particle expanded NK cells were cytotoxic against SKOV-3 cells. The cytotoxicity was at least partially the result of expression of the cytokine IFN-y and TNF-α which increased in production upon exposure to SKOV-3 cells and further still with additional stimulation through administration of IL-12 and/or IL-18. The induction of PD-L1 expression was observed in a variety of tumor cell lines exposed to IFNγ. Preclinical studies in humanized NSG-ovarian cancer bearing mice provide evidence that adoptively transferred PM21 NK cells and NK cells expanded with PM21-NK cells that secrete high amounts of IFN-γ induce PD-L1 on human ovarian tumor (Figure 3A). To further evidence that the PM21-NK or FC-NK cells were the specific inducers of PD-L1 on the tumors, the adoptively transferred NK cells were highly pure (99.99% NK cells) and contained virtually no T cells. Of the cells found in the peritoneum, PD-1 was expressed on T cells, but not NK cells (Figure 3B). Also, >99% of hCD45⁺ human lymphocytes in blood collected from mice after adoptive transfer were NK cells. To corroborate that the tumors are expressing PD-L1 when being attacked by PM21-NK or FC21-NK cells, a higher abundance of regulatory T cells (Tregs) were found in mice with the accumulation of ascites (Figure 3C). Tregs are known to be induced by PD-L1 and proliferate rapidly in response to PD-L1 expression on the tumors stimulated by IFNγ from NK cells. Although this immuno-suppressive induction of PD-L1 on tumors by PM21-NK cells helps tumor to escape the immune system, this mechanism could be advantageously utilized to induce the expression of a universal targetable antigen which would tag a large variety of tumor types (Figure 4). Treatment with humanized PD-L1 antibodies in conjunction with PM21-NK cells can unleash highly potent targeted anti-tumor response from NK cells by antibody-dependent cell-mediated cytotoxicity (ADCC) initiated by binding of anti-PD-L1 Ab to CD16 (IgG receptor FcγRIII) (Figure 4).

## Claims

1. An anti-PDL1 antibody and activated NK cells for use in treating a cancer or a viral, bacterial, fungal, or parasitic infection in a subject, wherein the activated NK cells are obtained by contacting a naive NK cell population with at least one or more of plasma membrane particles having membrane bound IL-21 (PM21 particles), exosomes having membrane bound IL-21 (EX21 exosomes) and feeder cells having membrane bound IL-21 (FC21 feeder cells).

2. The anti-PDL1 antibody and activated NK cells for use of claim 1, wherein the cancer is selected from lymphoma, B cell lymphoma, T cell lymphoma, mycosis fungoides, Hodgkin's Disease, myeloid leukemia, bladder cancer, brain cancer, nervous system cancer, head and neck cancer, squamous cell carcinoma of head and neck, kidney cancer, lung cancers such as small cell lung cancer and non-small cell lung cancer, neuroblastoma/glioblastoma, ovarian cancer, pancreatic cancer, prostate cancer, skin cancer, liver cancer, melanoma, squamous cell carcinomas of the mouth, throat, larynx, and lung, colon cancer, cervical cancer, cervical carcinoma, breast cancer, and epithelial cancer, renal cancer, genitourinary cancer, pulmonary cancer, esophageal carcinoma, head and neck carcinoma, large bowel cancer, hematopoietic cancers; testicular cancer; colon and rectal cancers, prostatic cancer, or pancreatic cancer.

3. The anti-PDL1 antibody and activated NK cells for use of claim 1 or 2, wherein the activation of NK cells by PM21 particles, EX21 exosomes, or FC21 feeder cells occurs for at least 1 to 21 days prior to the administration of the activated NK cells to the subject.

4. The anti-PDL1 antibody and activated NK cells for use of any one of claims 1 to 3, wherein the naive NK cells:
a. are sourced from peripheral blood, umbilical cord blood, or stem cells; or
b. are derived from stem cells or a NK cell like cell line, such as NK92.

5. The anti-PDL1 antibody and activated NK cells for use of any one of claims 1 to 4, wherein the activated NK cells are for administration between 1 and 21 days prior to the administration of the anti-PDL1 antibody.

6. The anti-PDL1 antibody and activated NK cells for use of any one of claims 1 to 4, wherein the anti-PDL1 antibody and the activated NK cells are for administration in a single composition.

7. The anti-PDL1 antibody and activated NK cells for use of any one of claims 1 to 6, wherein the NK cells are derived from autologous, haploidentical, or allogeneic NK cells.

8. The anti-PDL1 antibody and activated NK cells for use of any one of claims 1 to 7, wherein the NK cells have been contacted *ex vivo* with one or more of IL-12, IL-15, and IL-18.

9. The anti-PDL1 antibody and activated NK cells for use of any one of claims 1 to 7, wherein one or more of IL-12, IL-15, and IL-18 are for administration prior to, concurrently with, or following administration of the activated NK cells.

10. The anti-PDL1 antibody and activated NK cells for use of claim 9, wherein the one or more of IL-12, IL-15, and IL-18 is prepared for concurrent administration with the activated NK cells.

11. The anti-PDL1 antibody and activated NK cells for use of any one of claims 1 to 10, wherein the activated NK cells are expanded memory (XM) NK cells and are for use in combination with the anti-PDL1 antibody and PM21 particles and/or EX21 exosomes.

12. The anti-PDL1 antibody and activated NK cells for use to treat cancer of any one of claims 1 to 11, for use in combination with PM21 particles and/or EX21 exosomes.

13. The anti-PDL1 antibody and activated NK cells for use of any one of claims 1 to 12, wherein the activated NK cells are expanded cells and secrete higher amounts of IFN-γ than non-expanded NK cells.

14. The anti-PDL1 antibody and activated NK cells for use of any one of claims 1 to 13, wherein the FC21 feeder cells are NK cell stimulating feeder cells expressing membrane-bound IL-21, and the PM21 particles and EX21 exosomes are purified from FC21 feeder cells.

15. The anti-PDL1 antibody and activated NK cells for use to treat cancer of any one of claims 1 to 14, for use in combination with any one or more of a cancer therapeutic selected from a chemotherapeutic, an immunotherapeutic, a radiation therapy and a pain therapeutic.

## Patentansprüche

1. Anti-PDL1-Antikörper und aktivierte NK-Zellen zur Verwendung bei der Behandlung einer Krebserkrankung oder einer viralen, bakteriellen, Pilz- oder parasitären Infektion bei einem Subjekt, wobei die aktivierten NK-Zellen durch Kontaktieren einer naiven NK-Zellpopulation mit mindestens einem oder mehreren der Folgenden erhalten werden: Plasmamembranpartikel mit membrangebundenem IL-21 (PM21-Partikel), Exosomen mit membrangebundenem IL-21 (EX21-Exosomen) und Feederzellen mit membrangebundenem IL-21 (FC21-Feederzellen).

2. Anti-PDL1-Antikörper und aktivierte NK-Zellen zur Verwendung nach Anspruch 1, wobei der Krebs ausgewählt ist aus Lymphom, B-Zell-Lymphom, T-Zell-Lymphom, Mycosis fungoides, Morbus Hodgkin, myeloischer Leukämie, Blasenkrebs, Hirntumor, Krebs des Nervensystems, Kopf- und Halskrebs, Plattenepithelkarzinom des Kopf- und Halsbereichs, Nierenkrebs, Lungenkrebs wie kleinzelliger Lungenkrebs und nicht-kleinzelliger Lungenkrebs, Neuroblastom/Glioblastom, Eierstockkrebs, Bauchspeicheldrüsenkrebs, Prostatakrebs, Hautkrebs, Leberkrebs, Melanom, Plattenepithelkarzinome des Mundes, Rachens, Kehlkopfs und der Lunge, Dickdarmkrebs, Gebärmutterhalskrebs, Gebärmutterhalskarzinom, Brustkrebs und Epithelkrebs, Nierenkrebs, Urogenitalkrebs, Lungenkrebs, Speiseröhrenkarzinom, Kopf-Hals-Karzinom, Dickdarmkrebs, hämatopoetischen Krebserkrankungen, Hodenkrebs, Dickdarm- und Mastdarmkrebs, Prostatakrebs oder Bauchspeicheldrüsenkrebs.

3. Anti-PDL1-Antikörper und aktivierte NK-Zellen zur Verwendung nach Anspruch 1 oder 2, wobei die Aktivierung der NK-Zellen durch PM21-Partikel, EX21-Exosomen oder FC21-Feederzellen mindestens 1 bis 21 Tage vor der Verabreichung der aktivierten NK-Zellen an das Subjekt erfolgt.

4. Anti-PDL1-Antikörper und aktivierte NK-Zellen zur Verwendung nach einem der Ansprüche 1 bis 3, wobei die naiven NK-Zellen:
a. aus peripherem Blut, Nabelschnurblut oder Stammzellen gewonnen werden; oder
b. aus Stammzellen oder einer NK-Zell-ähnlichen Zelllinie wie NK92 stammen.

5. Anti-PDL1-Antikörper und aktivierte NK-Zellen zur Verwendung nach einem der Ansprüche 1 bis 4, wobei die aktivierten NK-Zellen zur Verabreichung zwischen 1 und 21 Tagen vor der Verabreichung des Anti-PDL1-Antikörpers vorgesehen sind.

6. Anti-PDL1-Antikörper und aktivierte NK-Zellen zur Verwendung nach einem der Ansprüche 1 bis 4, wobei der Anti-PDL1-Antikörper und die aktivierten NK-Zellen zur Verabreichung in einer einzigen Zusammensetzung vorgesehen sind.

7. Anti-PDL1-Antikörper und aktivierte NK-Zellen zur Verwendung nach einem der Ansprüche 1 bis 6, wobei die NK-Zellen von autologen, haploidentischen oder allogenen NK-Zellen stammen.

8. Anti-PDL1-Antikörper und aktivierte NK-Zellen zur Verwendung nach einem der Ansprüche 1 bis 7, wobei die NK-Zellen *ex vivo* mit einem oder mehreren von IL-12, IL-15 und IL-18 in Kontakt gebracht wurden.

9. Anti-PDL1-Antikörper und aktivierte NK-Zellen zur Verwendung nach einem der Ansprüche 1 bis 7, wobei ein oder mehrere von IL-12, IL-15 und IL-18 zur Verabreichung vor, gleichzeitig mit oder nach der Verabreichung der aktivierten NK-Zellen vorgesehen sind.

10. Anti-PDL1-Antikörper und aktivierte NK-Zellen zur Verwendung nach Anspruch 9, wobei das eine oder die mehreren von IL-12, IL-15 und IL-18 für die gleichzeitige Verabreichung mit den aktivierten NK-Zellen hergestellt sind.

11. Anti-PDL1-Antikörper und aktivierte NK-Zellen zur Verwendung nach einem der Ansprüche 1 bis 10, wobei die aktivierten NK-Zellen expandierte Gedächtnis-NK-Zellen (XM-NK-Zellen) sind und zur Verwendung in Kombination mit dem Anti-PDL1-Antikörper und PM21-Partikeln und/oder EX21-Exosomen vorgesehen sind.

12. Anti-PDL1-Antikörper und aktivierte NK-Zellen zur Verwendung zur Behandlung von Krebs nach einem der Ansprüche 1 bis 11, zur Verwendung in Kombination mit PM21-Partikeln und/oder EX21-Exosomen.

13. Anti-PDL1-Antikörper und aktivierte NK-Zellen zur Verwendung nach einem der Ansprüche 1 bis 12, wobei die aktivierten NK-Zellen expandierte Zellen sind und höhere Mengen an IFN-γ absondern als nicht expandierte NK-Zellen.

14. Anti-PDL1-Antikörper und aktivierte NK-Zellen zur Verwendung nach einem der Ansprüche 1 bis 13, wobei die FC21-Feederzellen NK-Zellen-stimulierende Feederzellen sind, die membrangebundenes IL-21 exprimieren, und die PM21-Partikel und EX21-Exosomen aus FC21-Feederzellen aufgereinigt werden.

15. Anti-PDL1-Antikörper und aktivierte NK-Zellen zur Verwendung zur Behandlung von Krebs nach einem der Ansprüche 1 bis 14, zur Verwendung in Kombination mit einem oder mehreren von einem Krebstherapeutikum, ausgewählt aus einem Chemotherapeutikum, einem Immuntherapeutikum, einer Strahlentherapie und einer Schmerztherapie.

## Revendications

1. Anticorps anti-PDL1 et cellules NK activées pour une utilisation dans le traitement d'un cancer ou d'une infection virale, bactérienne, fongique ou parasitaire chez un sujet, dans lesquels les cellules NK activées sont obtenues en mettant en contact une population de cellules NK naïves avec au moins une ou plusieurs particules de membrane plasmique présentant de l'IL-21 liée à la membrane (particules PM21), des exosomes présentant de l'IL-21 liée à la membrane (exosomes EX21) et des cellules nourricières présentant de l'IL-21 liée à la membrane (cellules nourricières FC21).

2. Anticorps anti-PDL1 et cellules NK activées pour une utilisation selon la revendication 1, dans lesquels le cancer est sélectionné parmi un lymphome, un lymphome à cellules B, un lymphome à cellules T, une mycose fongoïde, la maladie de Hodgkin, une leucémie myéloïde, un cancer de la vessie, un cancer du cerveau, un cancer du système nerveux, un cancer de la tête et du cou, un carcinome épidermoïde de la tête et du cou, un cancer du rein, des cancers du poumon tels que le cancer du poumon à petites cellules et le cancer du poumon non à petites cellules, un neuroblastome/glioblastome, un cancer de l'ovaire, un cancer du pancréas, un cancer de la prostate, un cancer de la peau, un cancer du foie, un mélanome, des carcinomes épidermoïdes de la bouche, de la gorge, du larynx et du poumon, un cancer du côlon, un cancer du col de l'utérus, un carcinome du col de l'utérus, un cancer du sein et un cancer épithélial, un cancer du rein, un cancer génito-urinaire, un cancer pulmonaire, un carcinome de l'œsophage, un carcinome de la tête et du cou, un cancer du gros intestin, des cancers hématopoïétiques ; un cancer des testicules ; des cancers du côlon et du rectum, un cancer de la prostate ou un cancer du pancréas.

3. Anticorps anti-PDL1 et cellules NK activées pour une utilisation selon la revendication 1 ou 2, dans lesquels l'activation des cellules NK par des particules PM21, des exosomes EX21 ou des cellules nourricières FC21 se produit pendant au moins 1 à 21 jours avant l'administration des cellules NK activées au sujet.

4. Anticorps anti-PDL1 et cellules NK activées pour une utilisation selon l'une quelconque des revendications 1 à 3, dans lesquels les cellules NK naïves :
a. proviennent du sang périphérique, du sang du cordon ombilical ou de cellules souches ;
ou
b. sont dérivées de cellules souches ou d'une lignée cellulaire de type cellules NK, telle que NK92.

5. Anticorps anti-PDL1 et cellules NK activées pour une utilisation selon l'une quelconque des revendications 1 à 4, dans lesquels les cellules NK activées sont destinées à être administrées entre 1 et 21 jours avant l'administration de l'anticorps anti-PDL1.

6. Anticorps anti-PDL1 et cellules NK activées pour une utilisation selon l'une quelconque des revendications 1 à 4, dans lesquels l'anticorps anti-PDL1 et les cellules NK activées sont destinés à être administrés dans une seule composition.

7. Anticorps anti-PDL1 et cellules NK activées pour une utilisation selon l'une quelconque des revendications 1 à 6, dans lesquels les cellules NK sont dérivées de cellules NK autologues, haplo-identiques ou allogéniques.

8. Anticorps anti-PDL1 et cellules NK activées pour une utilisation selon l'une quelconque des revendications 1 à 7, dans lesquels les cellules NK ont été mises en contact *ex vivo* avec un ou plusieurs parmi IL-12, IL-15 et IL-18.

9. Anticorps anti-PDL1 et cellules NK activées pour une utilisation selon l'une quelconque des revendications 1 à 7, dans lesquels un ou plusieurs parmi IL-12, IL-15 et IL-18 sont destinés à être administrés avant, simultanément ou après l'administration des cellules NK activées.

10. Anticorps anti-PDL1 et cellules NK activées pour une utilisation selon la revendication 9, dans lesquels les une ou plusieurs parmi IL-12, IL-15 et IL-18 sont préparées pour une administration simultanée avec les cellules NK activées.

11. Anticorps anti-PDL1 et cellules NK activées pour une utilisation selon l'une quelconque des revendications 1 à 10, dans lesquels les cellules NK activées sont des cellules NK à mémoire étendue (XM) et sont destinées à être utilisées en combinaison avec l'anticorps anti-PDL1 et des particules PM21 et/ou des exosomes EX21.

12. Anticorps anti-PDL1 et cellules NK activées pour une utilisation dans le traitement du cancer selon l'une quelconque des revendications 1 à 11, destinés à être utilisés en combinaison avec des particules PM21 et/ou des exosomes EX21.

13. Anticorps anti-PDL1 et cellules NK activées pour une utilisation selon l'une quelconque des revendications 1 à 12, dans lesquels les cellules NK activées sont des cellules développées et sécrètent des quantités plus élevées d'IFN-y que les cellules NK non développées.

14. Anticorps anti-PDL1 et cellules NK activées pour une utilisation selon l'une quelconque des revendications 1 à 13, dans lesquels les cellules nourricières FC21 sont des cellules nourricières stimulant les cellules NK exprimant l'IL-21 liée à la membrane, et les particules PM21 et les exosomes EX21 sont purifiés à partir de cellules nourricières FC21.

15. Anticorps anti-PDL1 et cellules NK activées pour une utilisation dans le traitement du cancer selon l'une quelconque des revendications 1 à 14, destinés à être utilisés en combinaison avec un ou plusieurs quelconques parmi un agent thérapeutique contre le cancer sélectionné parmi un agent chimiothérapeutique, un agent immunothérapeutique, une radiothérapie et un agent thérapeutique contre la douleur.
